# EUROPEAN PATENT APPLICATION

(11) **EP 1 821 094 A1**
(43) Date of publication of application: **22.08.2007**
(21) Application number: 05814552.5
(22) Date of filing: 07.12.2005
(51) Int. Cl.: G01N 15/14, C12Q 1/02, G01N 33/48

(54) **CELL MEASURING METHOD**

(30) Priority: 07.12.2004 JP 2004354593
(71) Applicant: Effector Cell Institute, Inc., Tokyo 153-0041 (JP)
(72) Inventor: NITTA, Nao, home, Meguro-ku, Tokyo 1530041; (JP)
(74) Representative: Hackney, Nigel John
(86) International application number: PCT/JP2005/022465
(87) International publication number: WO 2006/062131

(57) **Abstract**

A technology capable of measuring cell-related reactions at a time within a wide range of concentration. Cell groups (200) are located in a cell-storing space (110) filled with liquid (120). A specific substance is supplied to the liquid in the cell-storing space (110) to form in the liquid (120) the concentration gradient (131) of the specific substance (130)extending from one end (111) of the cell-storing space (110) toward the other end (112). The cell groups in the cell-storing space (110) is picked up by an imagining device (320) and captured into an information processor, and image obtained by the information processor is divided into a plurality of regions along the above concentration gradient, characteristic amounts are extracted from image portions in respective regions, and information indicating the interrelation between the extracted characteristic amounts and the concentration gradient is created.

## Description

### Technical Field

The present invention relates to a cell measuring method.

### Background Art

To develop drugs, test technologies, and the like, observation has been conducted using a microscope etc., on cells and various measurements thereof to collect various pieces of information on, for example, influences of drugs on cells. When such observation and measurement are conducted, in order to improve efficiency, an observation support system, located two dimensionally, is used, the system having a plurality of observation sites, which are minute sections for containing cells and observing them (hereinafter, referred to simply as "sites").

When observation, measuring, and the like, of cells are conducted using such an observation support system, time lapse photographing has been performed, in which the observation support system is mounted on an XYZ stage of a microscope, the stage is moved relative to an objective lens system in two-dimensions (XY) to sequentially place a plurality of sites in the field of view of the microscope, and the same target is photographed at predetermined time intervals. Because of this, in optical measuring devices using this type of microscope, the relative movement of the stage is precisely controlled so as to correctly place each site in the field of view of the microscope. Such a time lapse observation is described, for example, in a non-patent document "Shiro Kanegasaki et al., A novel optical assay system for the quantitative measurement of chemotaxis. Journal of Immunological Methods 282 (2003) 1 11" and in a patent document "JP-A-2002-277754".

When the reactivity, responsiveness, and the like of cells to chemical compounds are observed, it is required to measure differences in reactivity between kinds ofcells or variations in responsiveness in cases of changing the conditions of the cells.

In those cases, good results can be obtained by conducting experiments in suitable concentrations. For example, when it is desired to compare responsiveness of cells to a stimulation from cell to cell or it is desired to compare a variation in cell responsiveness under varied cell conditions, if the set concentration is too high or too low, difference between kinds of cells becomes hard to distinguish, so that good experimental results cannot be obtained.

Therefore, when responsiveness of cells to compounds is examined, it is necessary to set a suitable concentration of a compound stimulation. Upon setting the suitable concentration conditions, it is necessary to set a plurality of concentration conditions and conduct measurements of cell responses in respective concentrations. However, no consideration of this has been taken in the conventional art.

### Disclosure of the Invention

It is an object of the present invention to provide a technology capable of measuring cell-related reactions within a wide range of concentrations at one time.

According to a first aspect of the present invention, there is provided a method which measures cells by taking an image or images of cells using an imaging apparatus and capturing the image (s) into an information processing apparatus, to perform measurement on cells based on the obtained image (s) in the information processing apparatus, the method being characterized by including: filling a cell-storing space that stores the cells with a liquid; locating the cells in the cell-storing space; supplying a specified substance to the liquid in the cell-storing space to form a concentration gradient of the specified substance in the liquid from one end to another end of the cell-storing space; imaging the cells in the cell-storing space by the imaging apparatus to obtain (an) images of the cells and capturing the image(s) into the information processing apparatus; and generating information that indicates an interrelation between a characteristic amount of cells and the concentration of the specified substance.

According to a second aspect of the present invention, there is provided a method which measures cells by taking an image or images of cells using an imaging apparatus and incorporates the image(s) into an information processing apparatus, to perform measurement on cells based on the obtained image(s) in the information processing apparatus, the method being characterized by including: filling a cell-storing space that stores the cells with a liquid; locating the cells in the cell-storing space; supplying a specified substance to the liquid in the cell-storing space to form a concentration gradient of the specified substance in the liquid from one end to another end of the cell-storing space; imaging the cells in the cell-storing space by the imaging apparatus to obtain an image or images of the cells and capturing the image(s) into the information processing apparatus; and dividing, by the information processing apparatus, the obtained image into a plurality of regions along the concentration gradient and extracting a characteristic amounts from the image in each region; and generating, by the information processing apparatus, information that indicates an interrelation between the extracted characteristic amount and the concentration gradient.

According to a third aspect of the present invention, there is provided a method of measuring cells by using a cell observation support apparatus having a cell-storing region that stores cells to be observed, and a first reservoir space and a second reservoir space which sandwich the cell-storing region therebetween and communicate with each other through the cell-storing region, and when in use, each of the first reservoir space and the second reservoir space is filled with a liquid to fill the cell-storing region with the liquid, and an information processing apparatus that, when in use, obtains (an) image(s) of the cells stored in the cell-storing region and collects information related to the cells based on the obtained image (s), the method being characterized by including: locating the cells to be observed; filling the first reservoir space and the second reservoir space, and the cell-storing space with the liquid, and adding a cell stimulating substance that stimulates the cells, into the liquid in the first reservoir space; and then performing, by means of the imaging apparatus: processing of taking, a plurality of times, at different timing, images of the cells; processing of dividing the cell-storing region shown in the image taken each of the times, along a direction from the first reservoir side to the second reservoir side, into a plurality of sections, to count total number of cells in each section and to distinguish specified cells that exhibit a predetermined characteristic amount and count the number of the specified cells; processing of calculating, in each section, a ratio of the number of the specified cells that exhibit the characteristic amount to the total number of the cells counted in the section, based on the results of the counting; processing of generating information that indicates time course change of the ratio of the number of the specified cells that exhibit the characteristic amounts; and processing of outputting information that indicates the time course change, in response to an instruction.

### Brief Description of the Drawings

Fig. 1 is an explanatory diagram showing a principle of a cell measurement system according to an embodiment of the present invention;
Fig. 2 is a block diagram showing an example of a configuration of a cell measurement system that can be used when the cell measurement of the present invention is conducted;
Fig. 3 is a cross-sectional view showing a first example of an observation unit that can be used in the practice of the present invention;
Fig. 4 is a cross-sectional view showing a second example of an observation unit that can be used in the practice of the present invention
Fig. 5 is a cross-sectional view showing a third example of an observation unit that can be used in the practice of the present invention;
Fig. 6 is a cross-sectional view showing a fourth example of an observation unit that can be used in the practice of the present invention;
Fig. 7 is a cross-sectional view showing a fifth example of an observation unit that can be used in the practice of the present invention;
Fig. 8 is a graph showing results of measurement by fluorescence observation on the state of diffusion of FITC molecules after addition;
Fig. 9 is an explanatory diagram showing a case in which a characteristic that cells are degranulated due to a stimulating substance to change contrast is grasped;
Fig. 10 is an explanatory diagram showing a method which divides a screen on which an obtained image is displayed into a plurality of regions along a concentration gradient and extracting characteristic amounts;
Fig. 11 is a graph showing results of calculation of the proportion of degranulated cells at each point (section);
Fig. 12 is a graph showing results of measurements by adopting speed of cell movement in the concentration gradient direction as an index of strength of chemotactic activity and measuring the value of the speed of cell movement on each position of a channel; and
Fig. 13 is a graph showing some responsiveness of a cell with respect to stimulation.

### Best Mode for carrying out the Invention

Hereinafter, the best mode for carrying out the present invention is explained referring to the drawings. First, the principle of the present invention is explained and then specific application thereof is explained.

The present invention enables measurement within a wide range of concentrations at one time by forming a concentration gradient with good reproducibility on a plane on which many cells are present and measuring responses of cells at each position on the concentration gradient. As a result, according to the present invention, experiments necessary to search for an optimal cell stimulation concentration can be curtailed, thereby increasing the efficiency of the experimentation. Further, because the concentration of the chemical substance that stimulates the cells is set continuously depending on positions thereof, evaluations at an optimal concentration can be performed.

Regarding the method of analyzing data, the following is considered. That is, as described below, a captured image is sectioned into strip-like regions, a characteristic amount of cells contained in each region is obtained, and then analysis is performed.

### (a) Drug responsiveness evaluation method of cells under optimal concentration conditions (method of selecting optimal conditions)

When responsiveness of cells to drugs is measured, it is necessary to select suitable drug treatment concentrations for the cells and the drugs. Up to now, in such cases, some suitable concentrations have been selected and the properties of cells at each concentration have been independently measured to thereby select suitable concentrations. In these cases, to select a more suitable concentration, experiments must be repeated or performed in parallel, at a number of concentrations. In the technology of the present invention, however, continuous data on changes of the properties of cells, when the treatment concentration of the drug to the cell is continuously changed, can be obtained.

For example, in a case where two or more cell populations are present, and a difference in reactivity of each cell population to a chemical is to be studied, reactivity data at continuous drug concentrations for each cell population can be obtained at one time by the technology of the present invention. From the series of concentration conditions, the most appropriate concentration for evaluating a difference in the property of each cell population is selected, and at this concentration, comparison of responsiveness of each cell group to the drug at the most appropriate concentration can be performed. This technique makes unnecessary a series of experiments for deliberation regarding evaluating cell responsiveness to the drug at many concentrations, so that high quality data can be obtained with ease and reliably.

### (b) Obtaining function or value representing characteristic of responsiveness of cells to drugs

The responsiveness of cells to drugs can be continuously evaluated by the technique of the present invention. Based on "a series of concentration-responsiveness interrelation data" obtained here, a function or value that shows the characteristic of responsiveness of cells to drugs can be obtained. For example, when the reactivity of a cell to a drug is well expressed by a specified function of a concentration of the drug in some tests, by fitting the data obtained in the tests to the function appropriately, a function that approximates a concentration dependence of the response to the drug of each cell population tested can be obtained. Herein, the specified function is considered to include a sigmoid function or the like but is not limited thereto. It is also possible to obtain a concentration that corresponds to characteristic points such as a maximum, a minimum, or an inflection point from the function and adopt that value as a characteristic amount of the reactivity of the cell group to the drug.

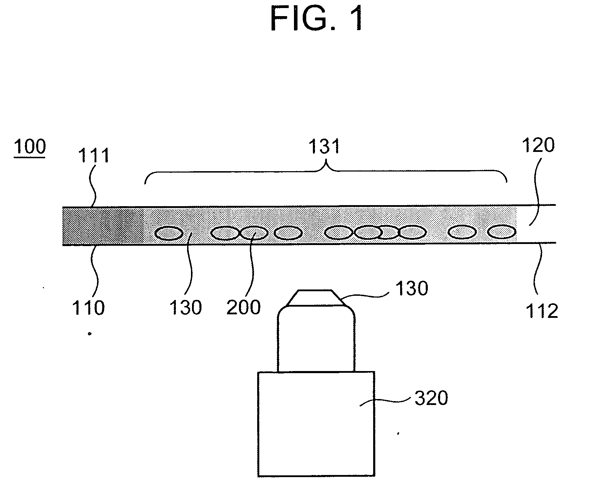

Then, an embodiment of the present invention will be explained by referring to Fig. 1. As shown in Fig. 1, in the embodiment of the present invention, a concentration gradient of a specified substance for observing the reaction of a cell thereto is formed in a space in which a plurality of cells are located. For this purpose, a cell-storing space 110 serving as a space in which cells are stored is formed in an observation unit 100. In Fig. 1, a structure of the cell-storing space 110 is shown schematically for illustrating the principle thereof. A more specific configuration will be described below.

The cell-storing space 110 is filled with a liquid 120 that is harmless to the cells. In the liquid, a plurality of cells 200 to be observed is located in a dispersed state. A substance for observing the reaction of the cells (hereinafter, referred to as a "stimulating substance") 130 is injected from one side of the cell-storing space 110 to form a concentration gradient 131 of the specified stimulating substance 130 in the cell-storing space 110. In this example, the state of the cells 200 is imaged by a digital camera (for example, CCD camera) 320 through a microscope 310 in the state where a solution having the stimulating substance 130 such as a compound dissolved therein is injected and the concentration gradient 131 of the specified stimulating substance 130 is formed in the cell-storing space 110. Note that, in this embodiment, a plurality of (for example, 12) cell-storing spaces 110 are formed on a silicon wafer using a photolithographic technology, and then the responsiveness of cells at each position of the concentration gradient in each cell-storing space 110 is measured. This method enables easy comparison of the actions of the compounds on the cell.

The responsiveness of cells to the stimulating substance varies depending on the concentration. Various cell phenomena such as, for example, gene expression, morphology change, release of physiologically active substances are conceivable as responses exhibited by cells. As a response to those, cells may exhibit some characteristic amounts that can be observed from outside. In the present invention, by utilizing this property, the characteristic amounts exhibited by the cells are extracted by analyzing the images thereof and information on the response of cells to the stimulating substance is obtained. In the case of the present invention, a concentration gradient is formed on the same screen. Therefore, no time-consuming trial and error, repeating experiments with varied concentrations, is necessary. Instead, responses at different concentrations can be observed by only one experiment.

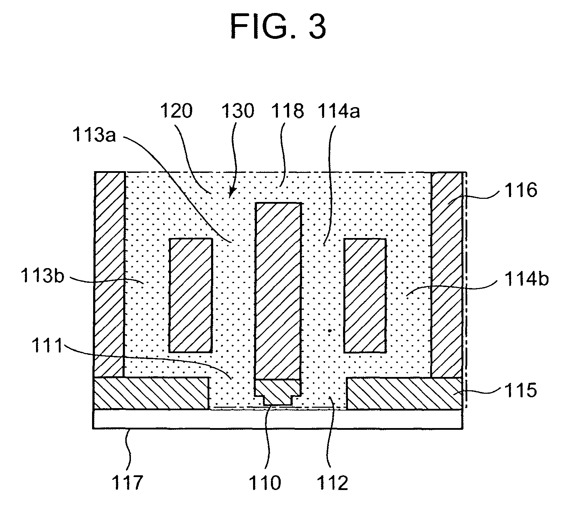

The cell-storing space 110 can specifically be realized in observation units 100 of various forms as shown in Figs. 3 to 7. The cell-storing space 110 is designed so as to locate the cells on a plane in a dispersed state so that observation thereof through a microscope or the like from the outside can be performed. Thus, a flat configuration is employed. The cell-storing space 110 has a thickness of, for example, about a size of the cell.

The observation unit shown in Fig. 3 includes a silicon wafer 115 having a flat engraving serving as the cell-storing space 110 formed thereon by using a photolithographic technology, a source region 111 positioned on one side of the cell-storing space 110 for flowing a stimulating substance thereinto, and an engraving serving as a drain space 112 positioned on the other side of the cell-storing space 110 for discharging the stimulating substance from the cell-storing space 110. The silicon wafer 115 is mounted on a glass substrate 117. This configuration allows the engraved space sandwiched between the silicon wafer 115 and the glass substrate 117 to constitute the cell-storing space 110, the source region 111, and the drain region 112. The cell-storing space 110 is formed as a flat space (i.e., channel) having a depth such that the cells are not superimposed on one another. Note that, the source region 111 and the drain region 112 are named for convenience to indicate the sides of injecting and discharging the stimulating substance. In cases of a symmetrical observation unit, each of the source region and the drain region may be on any side thereof.

On the upper surface of the silicon wafer 115 are provided reservoir constituting members 116 that constitute reservoirs. The reservoir constituting members 116 are made of, for example, a metal such as stainless steel. With the reservoir constituting members 116, a first reservoir space 113a and a third reservoir space 113b as well as a second reservoir space 114a and a fourth reservoir space 114b are formed. The first reservoir space 113a and the third reservoir space 113b communicate with the source region 111. On the other hand, the second reservoir space 112 and the fourth reservoir space 114 communicate with the drain region 112. Having this configuration allows the liquid 120 to flow between the first reservoir space 113a and the third reservoir space 113b, and the second reservoir space 114a and the fourth reservoir space 114b through the cell-storing space 110. However, in this embodiment, it is convenient for observation that the liquid itself does not create a stream, so a communication part 118 that enables the first reservoir space 113a and the third reservoir space 113b to communicate with the second reservoir space 114a and the fourth reservoir space 114b is provided on an upper part of the reservoir constituting members 116. By injecting the liquid 120 to reach the communication part 118, generation of a flow due to a pressure difference of the liquid 120 can be prevented.

The cells, stimulating substance, and the like are injected by using a syringe or the like. In this case, by placing the syringe in a state that the syringe reaches the source region 111, the cells, stimulating substance, and the like can be reliably injected to the cell-storing space 110.

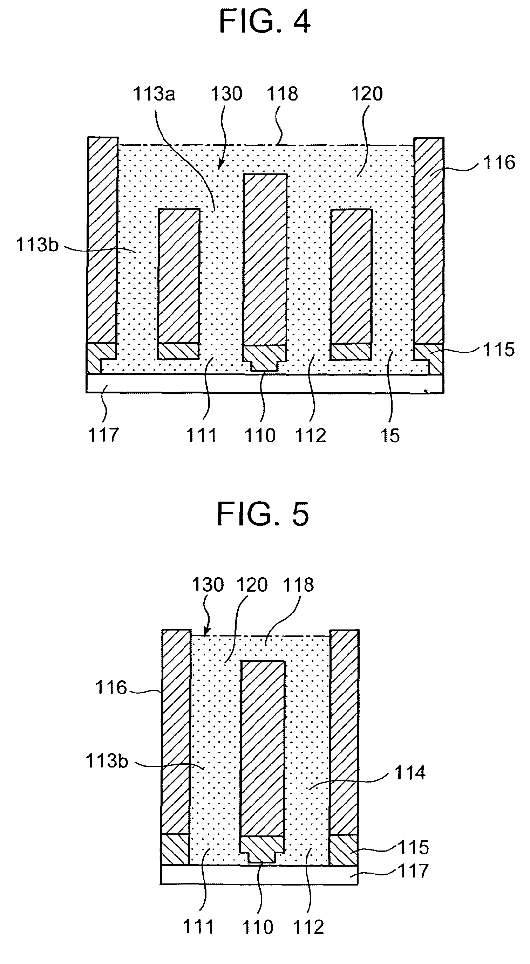

Fig. 4 shows an observation unit 100 having substantially the same structure as that shown in Fig. 3. The observation unit 100 has the same structure as the above-mentioned configuration shown in Fig. 3 except that it has a different engraving structure formed by the silicon wafer 115, and functions similarly.

The observation unit shown in Fig. 5 is of a structure that does not have the third reservoir space 113b and the fourth reservoir space 114b that are provided in the observation unit shown in Figs. 3 and 4. That is, it has a structure having a first reservoir space 113, a second reservoir space 114, the source region 111, the cell-storing space 110, and the drain region 112.

Note that, in the observation units 100 shown in Figs. 3 and 4, the third reservoir space 113b and the fourth reservoir space 114b are provided so as to absorb swinging of the liquid being injected. In particular, it is conceived that when the stimulating substance is injected by using a syringe or the like at a position apart from the source region 111, the third reservoir space 113b and the fourth reservoir space 114b serve to let out a part of the pressure generated upon pushing out the stimulating substance.

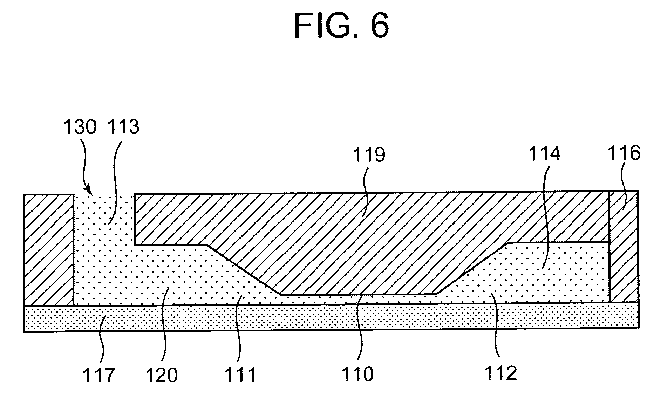

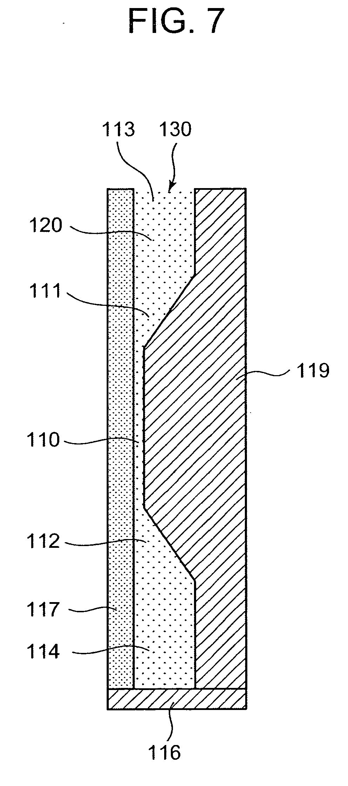

The observation units shown in Figs. 6 and 7 are of different types compared to those shown in Figs. 3 to 5. That is, the observation units shown in Figs. 6 and 7, and those shown in Figs. 3 to 5 differ in the configuration of the reservoir space and the manner of configuring the cell-storing space 110. The first reservoir space 113 is open to perform injection of the liquid, injection of the cells, injection of the stimulating substance, and the like therethrough. On the other hand, the second reservoir space 114 is not open. Further, stainless steel instead of silicon is directly used as a member 119 that constitutes the cell-storing space 110. This configuration simplifies the production of the observation unit, so that the effect of reducing the production cost can be obtained.

Fig. 6 shows an example of a transverse type, that is, a type to be placed horizontally. On the other hand, an example shown in Fig. 7 is a type to be placed vertically. In the example shown in Fig. 7, in the case of viscous cells, the cells stick to the glass substrate 117, so that the cells do not fall therefrom.

Here, a method of forming a concentration gradient is explained.

In the case of the observation unit shown in Fig. 5 described above, a communicating tube constituted by the cell-storing space (channel) 110, the first reservoir space 113, and the second reservoir space 114, in which the first reservoir space 113 and the second reservoir space 114 communicate through the cell-storing space (channel) 110. The communicating tube is filled with a suitable liquid. Further, in either one of the tubes of the first reservoir space 113 and the second reservoir 114, a solution containing a substance for forming a concentration gradient thereof is injected. This injection allows the substance to diffuse in the channel 110 from the source region 111 to the drain region 112. By filling the communication part 118 with the liquid 120, disturbance in the concentration gradient due to vigorous movement of the liquid in the channel 110 with influence of vibration or tilting can be reduced to a great extent. This enables maintenance of a stable concentration gradient.

As shown in Figs. 3 and 4, an observation unit in which the first reservoir space 113a is provided with the third reservoir space 113b as a branching pathway and the second reservoir space 114a is provided with the fourth reservoir space 114b as a branching pathway may be used. In this case, the liquid containing the target stimulating substance 130 is charged from the first reservoir space 113a or the second reservoir space 114a. Note that, when the liquid containing the stimulating substance 130 is injected from the side of the first reservoir space 113a, the stimulating substance diffuses from the source region 111 to the drain region through the channel 110. On the other hand, when the liquid containing the stimulating substance is charged from the side of the second reservoir space 114a, the drain region 112 shown in Figs. 3 and 4 serves substantially as a source region and the source region 111 on the opposite side serves as a drain region, substantially. Therefore, the source region and the drain region are names of convenience in the case of observation units shown in Figs. 3, 4 and 5.

Note that, a concentration gradient can also be formed by using the observation units shown in Figs. 6 and 7. In this case, the liquid containing the stimulating substance 130 is charged from the first reservoir space 113 that is open to form a concentration gradient of the stimulating substance 130 from the source region 111 to the drain through the channel 110.

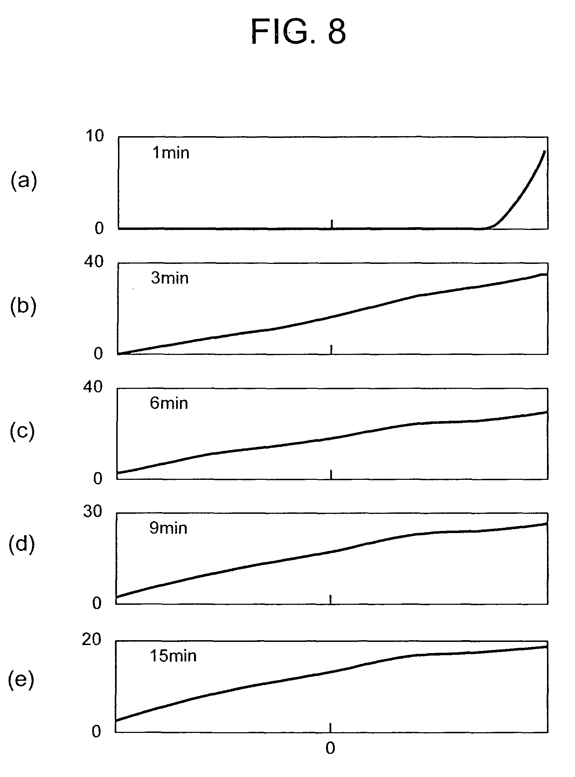

How the concentration gradient is formed is explained with reference to Fig. 8. Fig. 8 shows how diffusion occurs in a case where the observation unit shown in Fig. 4 is filled with the liquid and the specified stimulating substance 130 is charged thereto. In this experiment, FITC, which is a fluorescent substance, was added in the first reservoir space 113a. What are shown in Fig. 8 are the results of measurement of the state of diffusion of FITC molecule after addition by observing fluorescence. The horizontal axis of the graph indicates the position on the channel and the vertical axis thereof indicates the intensity of fluorescence. Here, there are shown concentration gradients of FITC molecules after 1, 3, 6, 9, and 15 minutes from the charging of the FITC molecules. It is understood that after 3 minutes, an approximately linear concentration gradient is formed and maintained.

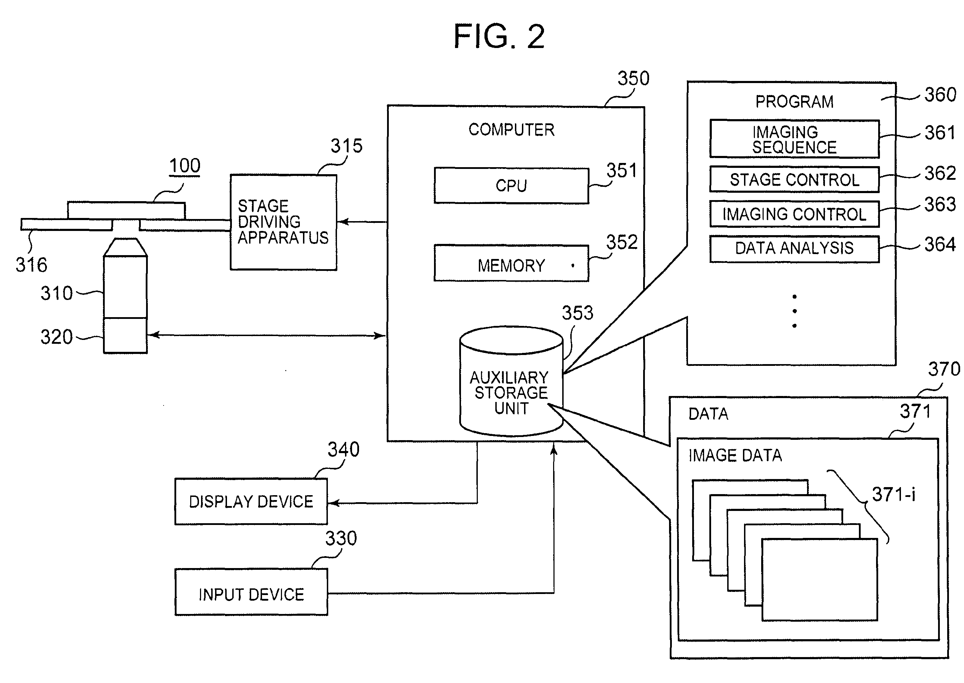

Fig. 2 shows an example of the configuration of the cell measurement system of the present invention. The cell measurement system shown in Fig. 2 includes an imaging apparatus for taking an image of cells stored in the cell-storing space 110 and an information processing apparatus for processing the taken image to obtain target information.

The imaging apparatus in this embodiment is constituted by a microscope 310, a CCD camera 320, a stage 316, an stage driving apparatus 315, and an information processing apparatus 350. Meanwhile, the information processing apparatus has an input device 330, a display device 340, and a computer 350.

The computer 350 has a central processing unit (CPU) 351, a memory 352, an auxiliary memory device 353. In the auxiliary memory device 353, an operation program 360 for the CPU 351 and data 370 are stored. The program 360 includes, in addition to an OS (not shown), an imaging sequence 361 for controlling measurement operations in a cell measurement system, a stage control 352 for controlling a stage, an imaging control 363 for controlling imaging, and a data analysis 364 for performing analysis of the obtained image data. Those programs are installed in the auxiliary memory device 353 from a memory medium, network, and the like. As the data, image data 371 is a typical example.

In imaging cells, cells for observation are provided in the above-mentioned observation units and measurement is performed under control of the computer 350. The measurement is performed according to the predetermined imaging sequence 361. That is, the computer 350 controls the driving of the stage driving apparatus 315 by the stage control program 362 such that a plurality of positions, for example, 12 points of the observation unit are imaged at a constant timing such as every other minute. The imaging sequence 361 instructs the CCD camera 320 to perform imaging of the cell group in the cell-storing space 110 at a constant timing by the imaging control program 363 with the positioning by the XY stage 316. Then, the images taken by the CCD camera 320 are captured into the memory 352 at a predetermined timing.

The captured image data are subjected to analysis processing by the data analysis program 64. First, the imaged data are stored in the auxiliary memory device 353 along with imaging conditions.

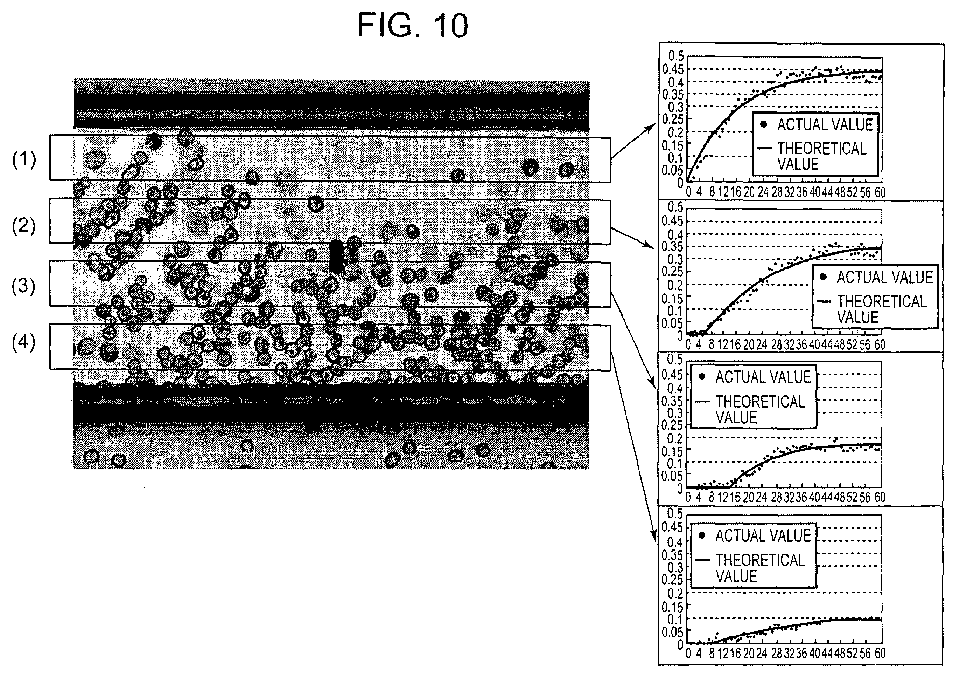

Thereafter, the obtained images are subjected to the designated processing. To extract characteristic amounts from the taken images, various methods can be used. For example, a screen of the obtained image may be sectioned into a plurality of regions along the concentration gradient and the characteristic amounts shown by the cell group in each section is extracted. The sections are defined as, for example, rectangles as shown in Fig. 10. The size of the section may be determined as appropriate. For example, there is a method that involves setting a frame on the screen that is displayed on a display device 340 by using an input device to have the section recognized by the computer 350. Alternatively, it is possible to adopt a configuration for determining sections by designating the number of the sections and uniformly dividing the screen into the designated number of sections. In any cases, to indicate, to a person, the section that the computer 350 recognizes, a rectangular frame can be displayed on the screen. Fig. 10 shows an example in which the frames are displayed.

Note that, in Fig. 10, numbers of 1 to 4 are attached to the left side of the frames showing the sections for convenience of explanation. In Fig. 10, images are shown with the upper side of the screen being the source side and the lower side being the drain side. Therefore, in the case shown in Fig. 10, the numerical values attached sequentially from with the upper side indicate decreasing order of concentration.

Here, the characteristic amounts shown by the cell groups include, for example, the following. As a matter of course, the characteristic amounts are not limited thereto.
(a) A ratio of the number of cells having a specified property in each region with respect to the total number of cells present in the region.
(a1) A case where the specified property is degranulation of cells.
(a2) A case where the cell is a mast cell in addition to the item (a1).
(b) An average value, a median, dispersion, standard deviation, and the like of a certain characteristic amount of the cell.
(b1) A case where a certain characteristic amount of the cell is movement speed of the cell.
(b2) A case where a certain characteristic amount of the cell is a specificity in the movement direction of the cell.

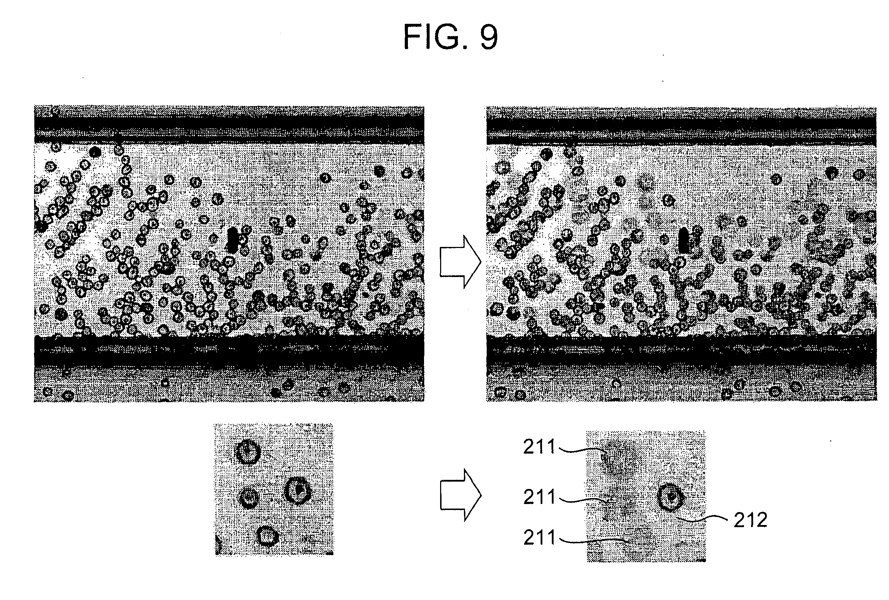

Extraction of the characteristic amount is performed by examining whether the cell is swelled or burst by using the contrast of images of the cells in the section as shown in Fig. 10. Besides, extraction of the characteristic amount may be performed by making a judgment in which the size of the cells such as average diameter or area is compared with predetermined threshold values and whether they exceed the threshold values is examined. Here, as shown in Fig. 9, a case is explained in which the characteristic, in which the cells are degranulated owing to the stimulating substance to change contrast, is perceived.

First, the number of cell groups is counted. That is, the cells are divided into a cell group having a high contrast (e.g., cell 212 shown in Fig. 9) and a cell group having a low contrast (e.g., cells 211 shown in Fig. 9), and each cell group is recognized to count the number of the cells. The recognition may be performed by various techniques. For example, a contour is determined and a closed contour is counted as one cell. In this case, the contrast is examined based on a difference in brightness between the contour line and the surrounding area. Alternatively, the images may be separated based on the high/low contrast by using a filter, and a contour line for each separated image is determined and the number of cells is counted. In any methods, the number of cells having high contrast and the number of cells having low contrast are counted.

Also, as an alternative to counting the number of cells, it is possible to measure the area on the screen occupied by the cell regions. For example, in the case where the cells are discriminated by using contrast as an index, the area of the region on the screen whose contrast is within a specified range of contrast is measured so that similar effects to those in the case where the number of cells is counted can be obtained. For example, when the ratio of cells having specified contrast is evaluated, a ratio of the area of the screen occupied by the cells having such the property to the area of the screen occupied by the total cells can be used as the ratio of the cells having such a property to the number of total cells.

The results of the measurement can be grasped, for example, as a ratio of degranulation due to a stimulating substance, by obtaining the ratio of the cells having low contrast to the number of total cells for each section. Further, when imaging is performed in a plurality of times at different timing, information that indicates response of cells to the stimulating substance can be generated at each concentration of the concentration gradient with lapse of time. Fig. 10 shows the characteristic amount (here, the ratio of the degranulated cells, that is, the activity intensity of the stimulating substance), which is exhibited by the cell group, based on the information thus generated at each concentration as a time course change. According to the information shown in Fig. 10, it can be understood that the activity differs depending on the concentration and the responsiveness varies with lapse of time.

### <Examples>

Next, the present invention is explained by way of examples in more detail.

Mast cells cause a reaction called degranulation to release granules retained in the cells, to outside the cells, owing to a certain compound stimulation. Upon the degranulation, the form and contrast of cells vary, so that the degranulated cells can be distinguished by an optical device. Here, a concentration gradient of a mast cell stimulating substance was formed in an environment in which mast cells were dispersed and the ratio of cells that caused degranulation at each position was calculated.

In performing experiments, TAXIScan (KK chamber), a tool for measuring chemotaxis of cells, was used (cf.: Kanegasaki, S et al., (2003), J. Immunol. Methods 282, 1-11). Into a gap between a microchannel and a glass substrate formed on a silicon wafer (cell-storing space 110), mast cells collected from the abdominal cavity of a rat, were introduced, and a mast cell stimulating agent as a stimulating substance was charged through the source region 111 connected to one end of the microchannel. Here, 1 microliter of a buffer solution containing 100 µM lysoPS and 2,000, 1,000, 200, or 80 ug/ml of concanavalin A was introduced. The degranulation of the mast cells was monitored by directly observing the cells in the channel on a microscope. The degranulation of the mast cells could be monitored by using a change in form, a change in contrast, or the like, as an index. Further, the degranulation could be automatically detected by image analysis by using a change in contrast or the like as an index.

The stimulating agent varies in concentration depending on positions on the screen since the stimulating agent forms a concentration gradient as a result of diffusion. By measuring the intensity of degranulation activity individually at each point on the screen, the intensity of the activity at the concentration on each point can be obtained (Fig. 10). The four graphs shown in the right side of Fig. 10 were obtained by measuring the ratio of the degranulated cells in the four areas in the photograph with lapse of time (the horizontal axis indicates time (minute) and the vertical axis indicates ratio (%)). The concentration gradient was formed by diffusion and the ratio of the degranulated cells became stabilized after a certain time. As stated above, the example shown in Fig. 10 had a concentration gradient such that the section (1) had the highest concentration and the sections (2), (3), and (4) had lower concentrations in the order.

Fig. 10 is a photograph of the cell-storing space 110 in which the mast cells are located and the stimulating agent was introduced from the upper part of the screen (left: before stimulation, right: after stimulation). It is confirmed that the cells on the upper part on the screen were degranulated to decrease the contrast, thereby being whitened. Note that reference numeral 211 indicates degranulated cells and reference numeral 212 indicates cells that were not degranulated.

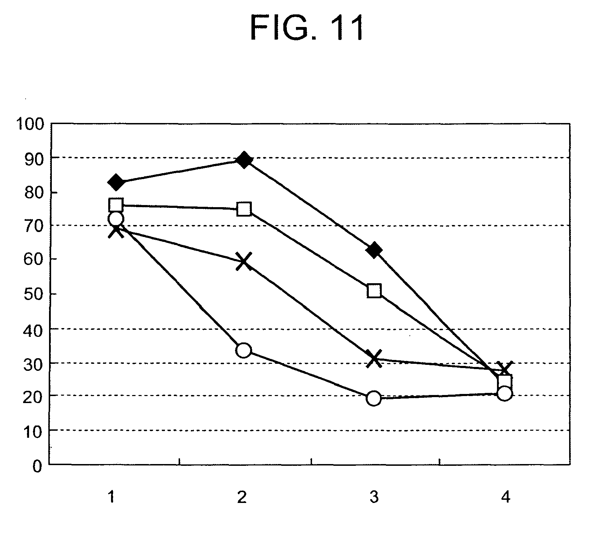

Fig. 11 is a graph illustrating results of calculation of the ratio of the degranulated cells at each point (section). The vertical axis indicates the ratio (%) of the degranulated cells and the horizontal axis indicates the concentration (µg/ml) of the stimulating agent. The numbers on the horizontal axis indicate the sections on the channel (cell-storing space) in which the concentration gradient has been formed. The concentration gradient is formed such that 1 designates a point having the highest concentration and the concentration is decreasing in the order of 1 to 4. Here, the numbers of 1 to 4 correspond to the section numbers (1) to (4) attached to Fig. 10. Note that the concentration of concanavalin A used as a stimulating agent was 2, 000 µg/ml for solid squares, 1, 000 µg/ml for open squares, 200 µg/ml for symbols x, and 80 µg/ml for open circles. The graph indicates that the ratio of degranulated cells on the points 2 and 3 varies depending on the concentration of the stimulating agent and the values correlate with the concentrations of the stimulating agent.

The graph shown in Fig. 11 shows substantially no difference in concentration of the stimulating agent at the sections (1) and (4) while the sections (2) and (3) indicate a clear difference in reaction depending on the concentration. For example, in the experiments in which fluctuation of activity of the cells to the intensity of stimulation is examined or an inhibitory/promoting effect of a reagent given to the activity of the cells is confirmed, appropriate data can be obtained by adopting the concentration of the stimulating agent corresponding to the region of the sections (2) and (3). In this manner, use of the concentration gradient enables observation of cell responses at continuously varying concentrations in one experiment, so that comparison under optimal conditions is possible.

Then, another example is explained. Here, concentration-dependent change of the chemotaxis activity of the cells is described.

Also the concentration-dependent change of the chemotaxis activity of the cells can be studied by taking into consideration the position of the cells on the concentration gradient. The chemotaxis of a cell is a function of the cell to sense a concentration gradient of a specified factor and move in the direction of higher concentration. The chemotaxis activity is known to exhibit a bell-shaped activity which exhibits the highest activity at a specified concentration and which is decreased at concentrations higher or lower than the specified concentration. By the conventional method, it can be confirmed, by performing experiments at a number of concentrations, that an activity with respect to concentrations is bell-shaped. By utilizing the fact that the concentration differs at each position of the concentration gradient environment, the concentration characteristics of the activity can be found with ease. To confirm the characteristics with experiments, TAXIScan (KK chamber), a tool for measuring chemotaxis of cells, was used (cf., Kanegasaki S et al., (2003) J. Immunol. Methods 282, 1-11 for the method and material).

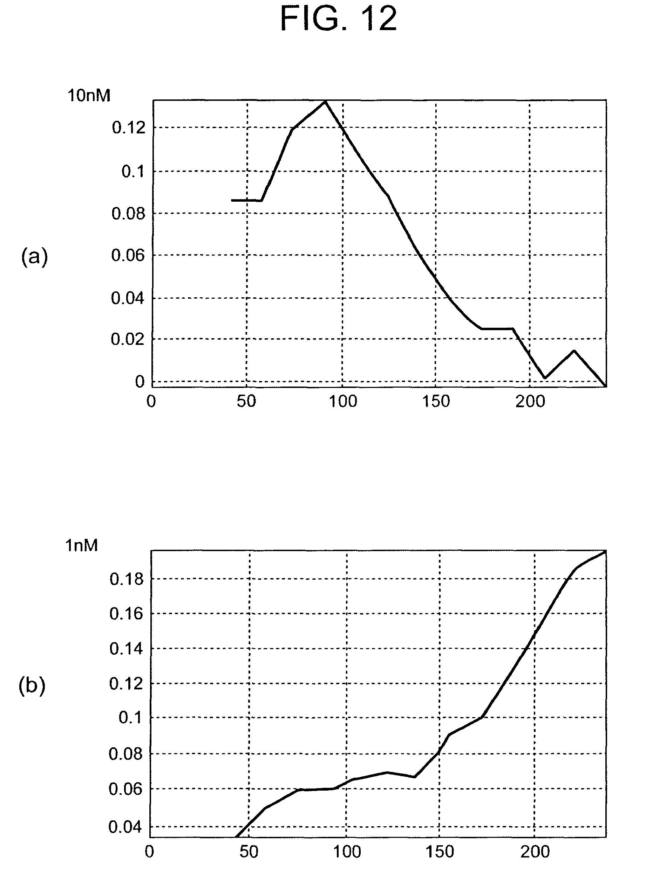

Here, Jurkat cells were introduced into the cell-storing space 110 and a concentration gradient of SDF-1a was formed therein and the movement of the cells was measured. The concentration of SDF-1a used when the concentration gradient was formed was of three kinds, i.e., 10 nM and 1 nM, and 1 microliter of the liquid was injected for each concentration. Since the Jurkat cells indicated chemotaxis in the direction of the concentration gradient of SDF-1a, the movement speed of the cells in the direction of the concentration gradient was adopted as an index for the intensity of chemotaxis activity and the value thereof was measured on each position on the channel (cell-storing space 110). Fig. 12 shows the obtained results. The horizontal axis of the graph indicates position on the channel and the vertical axis indicates chemotaxis activity (the component of the cell travelling speed in the direction of the concentration gradient). The concentration gradient was formed starting from the right-hand side of the graph, so the concentration is higher as the position is more shifted toward the right-hand side. The vertical axis indicates the speed (µm/sec) of the cell movement in the direction of the concentration gradient. When the concentration of SDF-1a added was 1 nM, the chemotaxis activity increased at increased concentration as shown in Fig. 12(b). The results obtained when the concentration of SDF-1a was 10 nM indicate that a peak of activity was present at a point in the midway of the channel shown in Fig. 12(a) and at a higher concentration than that at that point the chemotaxis activity was decreasing. This indicates that the chemotaxis activity is certainly a bell-shaped activity.

The measurement of cell movement can be performed, for example, by dividing the screen shown in Fig. 10 into a plurality of meshes, focusing on a portion of the contour line of a cell in the mesh, measuring how the position of the contour line is displaced with time, and averaging the x components and y components of the displacements to obtain an average speed from an average direction of displacement and timing of imaging.

As described above, in the present invention, cell groups are charged in a cell-storing space, and at the same time, a stimulating substance is injected to the cell groups to form a concentration gradient of the stimulating substance, images of the cells are taken, the obtained images are divided into sections along the concentration gradient, and characteristic amounts of the cell group in each section are extracted based on the processing of the images. The measurements that must be performed a number of times for each concentration and for each time in the conventional technology can be performed by a single processing.

That is, when differences in reactivity between the kinds of the cells or when variation in responsiveness in case of changing the conditions of the cells is measured, good measurement results can be obtained by performing experiments at appropriate concentrations. For example, when it is desired to make comparison on responsiveness of cells to certain stimulation between the kinds of the cells or comparison on changes in responsiveness of cells under varied conditions, concentration set at a too high level or a too low level makes differences between the kinds of the cells obscure, and good experimental results cannot be obtained.

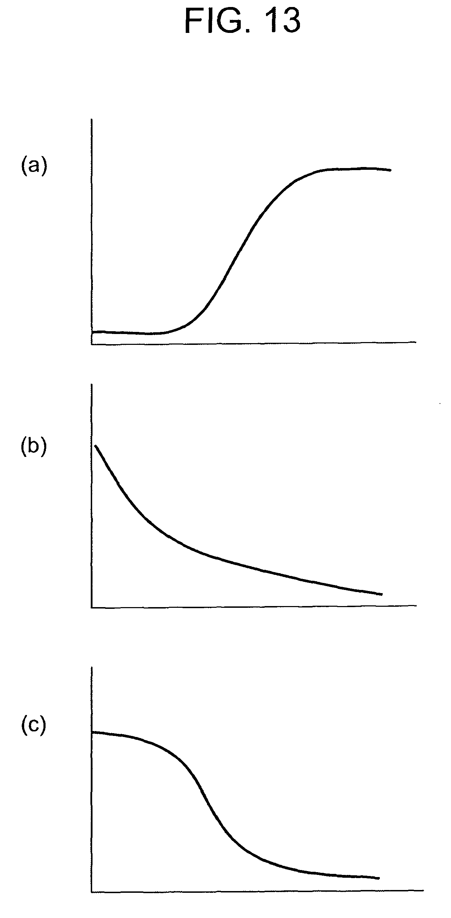

Here, assume that any responsiveness to the stimulation to the cells is expressed as shown in Fig. 13(a) (horizontal axis: concentration, vertical axis: activity). Then, when a concentration gradient is formed in an environment where cells are suitably dispersed as shown in Fig. 13 (b) (horizontal axis: position, vertical axis: concentration), the responsiveness of the cells at each position in the concentration gradient is shown in Fig. 13 (c) (horizontal axis: position, vertical axis: activity). In general experiments, in cases of testing a number of concentrations, the concentration is varied stepwise and reaction at each concentration is observed, so that the concentrations to be tested are discontinuous. Further, to confirm the reaction at a number of concentrations, a corresponding number of tests must be performed independently. However, by utilizing a concentration gradient, cell responses at each concentration in a continuous concentration range can be confirmed by a single independent test. This reduces the number of independent tests, thus making the experiment easier.

Note that evaluation of the activity of cells is applicable to all life phenomena that can be evaluated on a microscope such as morphological change or motility of cells, localization change of intracellular molecules using a fluorescent probe, and gene expression.

## Claims

1. A method which measures cells by taking an image or images of cells using an imaging apparatus and capturing the image (s) into an information processing apparatus, to perform measurement of cells based on the obtained image(s) in the information processing apparatus, the method comprising:
filling a cell-storing space that stores the cells, with a liquid;
locating the cells in the cell-storing space;
supplying a specified substance to the liquid in the cell-storing space to form a concentration gradient of the specified substance in the liquid from one end to another end of the cell-storing space;
imaging the cells in the cell-storing space by the imaging apparatus to obtain (an) image(s) of the cells and capturing the image(s) into the information processing apparatus; and
generating information that indicates an interrelation between the concentration of the specified substance and a characteristic amount of cells from the obtained image(s).

2. A method of measuring cells according to claim 1, wherein the concentration gradient is formed using a fluorescent substance and is measured by observation of the fluorescence.

3. A method of measuring cells according to claim 1 or 2, wherein the characteristic amount of the cells is any one of the degranulation, movement speed, specificity in movement direction, size, diameter, area, contrast, morphological change, localization change of intracellular molecules, and gene expression, of the cells.

4. A method of measuring cells according to claim 3, wherein the characteristic amount of cells is a characteristic amount that is obtained by determining a contour of the cells from the image (s) and distinguishing a closed object as one cell, thus obtaining the characteristic amount from the distinguished cells.

5. A method which measures cells by taking (an) image (s) of cells using an imaging apparatus and capturing the image (s) into an information processing apparatus, to perform measurement of cells based on the obtained image(s) in the information processing apparatus, the method comprising:
filling a cell-storing space that stores the cells, with a liquid;
locating the cells in the cell-storing space;
supplying a specified substance to the liquid in the cell-storing space to form a concentration gradient of the specified substance in the liquid from one end to another end of the cell-storing space;
imaging the cells in the cell-storing space by the imaging apparatus to obtain (an) image(s) of the cells and capturing the image(s) into the information processing apparatus; and
dividing, by the information processing apparatus, the obtained image(s) into a plurality of regions along the concentration gradient and extracting a characteristic amount from the image(s) in each region; and
generating, by the information processing apparatus, information that indicates an interrelation between the extracted characteristic amount and the concentration gradient.

6. A method of measuring cells according to claim 5, wherein the characteristic amount is extracted by a difference in contrast shown by the image(s) of the cells.

7. A method of measuring cells according to claim 6, wherein a ratio of the number of cells having a specified property in each region, to the number of cells overall in each the region is extracted from the image(s) for each region as the characteristic amount.

8. A method of measuring cells according to claim 7, wherein the specified property is the degranulation of cells.

9. A method of measuring cells according to claim 8, wherein the cells are mast cells.

10. A method of measuring cells according to claim 6, wherein any one of indices, selected from average value, median, dispersion, and standard deviation concerning movement of whole cells located in each of the regions, is extracted from the image(s) as the characteristic amount.

11. A method of measuring cells according to claim 10, wherein what is selected as the characteristic amount is an index concerning movement speed of the cells.

12. A method of measuring cells according to claim 10, wherein what is selected as the characteristic amount is an index concerning specificity of the movement direction of the cells.

13. A method which measures cells by using a cell observation support apparatus having a cell-storing region that stores cells to be observed, and a first reservoir space and a second reservoir space which sandwich the cell-storing region therebetween and communicate with each other through the cell-storing region, and when in use, each of the first reservoir space and the second reservoir space is filled with a liquid to fill the cell-storing region with the liquid, and an information processing apparatus that, when in use, obtains (an) image(s) of the cells stored in the cell-storing region and collects information related to the cells based on the obtained image(s), the method comprising:
locating the cells to be observed;
filling the first reservoir space and the second reservoir space, and the cell-storing space with the liquid, and adding a cell stimulating substance that stimulates the cells, into the liquid in the first reservoir space; and then
performing, by means of the imaging apparatus:
processing of taking, a plurality of times, at different timing, images of the cells;
processing of dividing, into a plurality of sections, the cell-storing region shown in the image taken each of the times, along a direction from the first reservoir side to the second reservoir side, to count total number of cells in each section and to distinguish specified cells that exhibit a predetermined characteristic amount and count the number of the specified cells;
processing of calculating, in each section, a ratio of the number of the specified cells that exhibit the characteristic amount to the total number of the cells counted in the section, based on the results of the counting;
processing of generating information that indicates time course change of the ratio of the number of the specified cells that exhibit the characteristic amount; and
processing of outputting information that indicates the time course change, in response to an instruction.
